# EUROPÄISCHE PATENTSCHRIFT

(11) **EP 3 119 931 B1**
(45) Veröffentlichungstag und Bekanntmachung des Hinweises auf die Patenterteilung: **21.04.2021**
(21) Anmeldenummer: 15710478.7
(22) Anmeldetag: 13.03.2015
(51) Int. Cl.: D01F 1/10, D01F 2/00, C08K 5/00

(54) **FASERN UND GARNE MIT OKKLUSIONSFUNKTION**
FIBRES AND YARNS WITH BLOCKING FUNCTION
FIBRES ET FILS À FONCTION OCCLUSIVE

(30) Priorität: 19.03.2014 DE 102014004258
(43) Veröffentlichungstag der Anmeldung: 25.01.2017
(60) Teilanmeldung: 19208767.4
(73) Patentinhaber: Bauerfeind AG, 07937 Zeulenroda (DE)
(72) Erfinder: BAUERFEIND, Hans B., 07937 Zeulenroda-Triebes (DE)
(74) Vertreter: Wohlfahrt, Jan Günther
(86) Internationale Anmeldenummer: PCT/EP2015/055318
(87) Internationale Veröffentlichungsnummer: WO 2015/140072

(56) Entgegenhaltungen:
- WO-A2-2009/133059
- CN-A- 102 995 295
- CN-A- 103 225 172
- GB-A- 2 414 394
- JP-A- H 092 960
- JP-A- H08 325 831
- US-A- 5 232 769
- US-B2- 7 422 712

## Beschreibung

Die vorliegende Erfindung betrifft eine Polymerzusammensetzung, enthaltend ein Polymer und mindestens einen Wirkstoff, Formkörper mit einer solchen Polymerzusammensetzung, die Verwendung der Formkörper und Polymerzusammensetzungen und entsprechende Garne, Vliesstoffe, Kleidungsstücke und medizinische Hilfsmittel.

Polymerzusammensetzungen, die durch Additive "funktionalisiert" sind, sind bekannt. So beschreibt die US-A 5,766,746 beispielsweise Cellulosefasern, die eine flammwidrige Komponente enthalten. Die DE 100 37 983 A1 beschreibt Polymerzusammensetzungen mit einem Gehalt an Alkaloid und die DE 100 07 794 A1 Polymerzusammensetzungen mit einem Material aus Meerespflanzen oder-tieren.

Solche Polymerzusammensetzungen werden zu Garnen, textilen Flächen und Kleidungsstücken weiterverarbeitet.

Die CN 103 225 172 A offenbart Chondroitinsulfat-Nanofaservliesgewebe und ein Herstellungsverfahren und medizinische Zwecke davon. Das Nanofaservliesgewebe umfasst Chondroitinsulfat und synthetische Makromoleküle und verwendet hauptsächlich die Elektrospintechnik, um die gemischte Lösung des Chondroitinsulfats und des synthetischen Makromoleküls zu Nanofasern zu verarbeiten.

Die CN 102 995 295 A offenbart Panax Pseudo-Ginseng nicht-gewebte Stoffe und Herstellungsverfahren dieser. Die Stoffe offenbart gemäß CN 102 995 295 A sind durch ultrafeine Fasern mit einem Durchmesser von 60-500 nm erhalten durch Elektrospinning sowie einer Dicke des Films, gebildet durch die ultrafeinen Fasern, von 10-50 µm gekennzeichnet. Die Stoffe sollen als Verbandsmaterial verwendet werden können.

Die US 5 232 769 A offenbart textile Strukturen mit Mikrokapseln welche durch einen Klebstoff an die textilen Strukturen gebunden sind, wobei die Mikrokapseln einen Durchmesser in einem Bereich von 2-40 µm aufweisen sowie eine Wandmembran umfassend ein synthetisches hoch molekulares Material und umschließend eine Substanz zur Verbesserung des physiologischen Zustands der menschlichen Haut, wobei die Substanz ausgewählt ist aus der Gruppe bestehend aus Ascorbinsäure, Tocopherole, Algenextrakt, Antipruritika und Analgetika, wobei Mikrokapseln und Klebstoff in einem Gewichtsverhältnis in einem Bereich von 10:1-1:5 vorliegen, die Gesamtmasse von Mikrokapseln und Binder 0,3-15 % bezogen auf das Gewichtsverhältnis der Fasern mit denen die Mikrokapseln und der Klebstoff verbunden sind.

Die JP H08-325831 A offenbart regenerierte Cellulosefasern mit Terpenoiden und Flavonoiden gekennzeichnet dadurch, dass die Fasern mindestens ein Extrakt einer Pflanze enthalten, welches einen oder zwei oder mehrere Glykoside von Anthron enthält.

Die JP H09-2960 A offenbart ein Hautbehandlungsmaterial aus Tanninen absorbiert und fixiert an Faseraggregaten.

Die GB 2 414 394 A offenbart die Verwendung eines nicht ionischen Surfactants in der Herstellung eines Medikaments für die Behandlung von vaginalen Geruch, wobei der nicht ionische Surfactant auf einen Tampon aufgetragen ist.

Die US 7 422 712 B2 offenbart ein Verfahren zur Einbringung eines flüssigen Additiv in ein nicht gewebtes Gewebe, wobei das flüssige Additiv in Füllpartikel geladen wird, um eine trockene flüssige Konzentrat zu bilden.

Die WO 2009/133059 A2 offenbart eine Nanofasermatrix umfassend ein aktives Agens suspendiert oder eingewickelt in Fasern gebildet durch Elektrospinning von einem oder mehreren verzweigten Polymeren und wobei die Nanofasermatrix weniger als 0,1 Gewichtsprozent organischen Lösungsmittels umfasst und wobei die verzweigten Polymere ein Molekulargewicht von 1000-100.000 g/Mol und eine Schmelztemperatur von 30° oder höher aufweisen.

Der Zusatz von Additiven zur Funktionalisierung von Polymerfasern führt oftmals zu Problemen, die bei der Verwendung der Fasern notwendigen Eigenschaften, insbesondere mechanische Festigkeit, Schlingenfestigkeit, Faserdehnung, Scheuereigenschaft und Anfärbbarkeit, dennoch zu erhalten.

Es besteht aber eine stetige Nachfrage nach funktionellen Fasern und Kleidungsstücken aus solchen Fasern mit spezifischen Additiven, so dass es wichtig ist, solche Fasern bereitzustellen, bei denen die beschriebenen Schwierigkeiten überwunden werden, damit funktionalisierte Fasern die entsprechenden Anforderungen an das aus ihnen produzierte Kleidungsstück erfüllen können.

Für den Markt besonders relevante Additive sind dabei Additive mit einer Wirkung auf den Organismus des Trägers der aus den Polymerzusammensetzungen hergestellten Kleidungsstücke.

Bisher bekannte Strukturen beinhalten nur geringe Konzentrationen dieser Stoffe. Diese Stoffe werden durch Tränkung oder Beschichtung auf die Trägermatrix aufgetragen. Dieses führt beispielsweise zu geringen Waschbeständigkeiten, sodass solche Stoffe regelmäßig zur Aufrechterhaltung ihrer Wirkweise neu zugeführt werden müssen.

Daher liegt der vorliegenden Erfindung das technische Problem zugrunde, eine Polymerzusammensetzung bereitzustellen, die ein entsprechendes Additiv enthält, wobei dennoch eine gute Stabilität und Verarbeitbarkeit der aus der Polymerzusammensetzung hergestellten Formkörper resultiert.

Bei eng an der Haut anliegenden Kleidungsstücken und medizinischen Hilfsmitteln, beispielsweise Bandagen und Kompressionsstrümpfen, spielt beim Tragekomfort eine Regulierung der Hautfeuchtigkeit eine Rolle, insbesondere sollte ein Austrocknen der Haut unterbunden werden. Auch sollen solche textile Flächen nicht zu Hautaustrocknung und zu Hautreizungen führen oder bereits bestehende Hauterkrankungen wie Neurodermitis verstärken. Dabei soll auch ein mit solchen Hautkrankheiten verbundener Juckreiz oder Schmerz zumindest verringert werden.

Das der vorliegenden Erfindung zugrunde liegende technische Problem ist auch die Bereitstellung von insbesondere in dieser Hinsicht verbesserten Kleidungsstücken und medizinischen Hilfsmitteln und deren Ausgangsmaterial.

Die vorliegende Erfindung löst das ihr zugrunde liegende technische Problem durch den Gegenstand der unabhängigen Ansprüche.

Die vorliegende Erfindung löst das ihr zugrunde liegende technische Problem durch ein Garn, enthaltend einen Formkörper umfassend eine Polymerzusammensetzung, enthaltend ein Polymer und mindestens einen Wirkstoff ausgewählt aus der Gruppe bestehend aus einem eine Okklusion innere Okklusion erzeugenden Wirkstoff oder ei-nem eine äußere Okklusion erzeugenden Wirkstoff und Mischungen davon, wobei das Polymer ausgewählt ist aus der Gruppe bestehend aus Cellulose, modifizierter Cellulose, Modifikationen davon, Derivaten davon und Mischungen davon.

Offenbart wird eine Polymerzusammensetzung, enthaltend ein Polymer und mindestens einen Wirkstoff ausgewählt aus der Gruppe bestehend aus einem eine Okklusion erzeugenden Wirkstoff, insbesondere einem eine innere Okklusion erzeugenden Wirkstoff oder einem eine äußere Okklusion erzeugenden Wirkstoff, einem feuchtigkeitsspendenden Wirkstoff, einem Schmerz oder Juckreiz reduzierenden Wirkstoff und Mischungen davon.

Die Polymerzusammensetzung enthält also erfindungsgemäß neben dem Polymer zusätzlich mindestens einen Wirkstoff, wobei es sich bei dem Wirkstoff um einen eine innere Okklusion erzeugenden Wirkstoff oder einen eine äußere Okklusion erzeugenden Wirkstoff handeln kann. Natürlich sind auch das Vorhandensein von zwei oder mehreren dieser Wirkstoffe, insbesondere auch mit einen feuchtigkeitsspendenden Wirkstoff oder einen Schmerz oder Juckreiz reduzierenden Wirkstoff, und Mischungen dieser Wirkstoffe von der Erfindung erfasst.

Es zeigte sich überraschender Weise, dass solche Wirkstoffe in eine Polymerzusammensetzung eingebracht werden können und dort als Additiv ihre Wirkung, insbesondere direkt auf der anliegenden Haut entfalten können, wenn die Polymerzusammensetzung in Form von entsprechenden Produkten wie Kleidungsstücken und medizinischen Hilfsmitteln an der Haut anliegt. Insbesondere können diese Wirkstoffe dabei in vorteilhafter Weise ein Austrocknen der Haut verhindern und/oder Schmerz und Juckreiz bei Krankheiten wie Neurodermitis zumindest vermindern. Auch können die Wirkstoffe dazu verwendet werden, weitere Wirkstoffe in die Haut eindringen zu lassen.

Dabei konnte überraschender Weise auch festgestellt werden, dass die in der Polymerzusammensetzung als Additive enthaltene erfindungsgemäße Gruppe von Wirkstoffen über den Anwendungszeitraum eine gute Beständigkeit aufweist und somit über diesen Zeitraum ihre Wirkung entfalten kann.

Es zeigte sich darüber hinaus überraschenderweise, dass die offenbarten Polymerzusammensetzungen, insbesondere, wenn sie Cellulose enthalten beziehungsweise Cellulose-basiert sind, zu Fasern führen, die trotz des Zusatzes mindestens einem der erfindungsgemäßen Additive dieselben hervorragenden Eigenschaften zeigen, wie reine Fasern, insbesondere Cellulosefasern, beispielsweise bezüglich ihrer Feinheit, Reißkraft, Reißkraftvariation, Dehnung, Nassdehnung, feinheitsbezogenen Reißkraft, feinheitsbezogenen Nassreißkraft, feinheitsbezogenen Schlingenreißkraft, Nassscheuerungsvariation und Nassmodulen. Dennoch zeigten diese Fasern gleichzeitig die durch das mindestens eine Additiv verliehenen positiven Eigenschaften.

Bevorzugt werden die Fasern aus einer Spinnlösung hergestellt. Es zeigte sich, dass trotz der Zugabe mindestens eines erfindungsgemäßen Additivs überraschenderweise keine Zersetzung einer Spinnlösung, insbesondere, wenn sie Cellulose enthält, und ganz besonders wenn die Spinnlösung aus Cellulose, N-Methylmorpholin-N-Oxid (NMMNO) und Wasser als Hauptkomponenten gebildet wird.

Geeignete Herstellverfahren, insbesondere für Polymerzusammensetzungen, die Cellulose oder modifizierte Cellulose enthalten, und insbesondere in Form von Formkörpern sind aus der DE 10 2007 054 702 A1 bekannt.

Die Polymerzusammensetzung kann Polymere enthalten, wie sie typischer weise für die Herstellung von Fasern, Filamenten, Garnen, Vliesstoffen, Kleidungsstücken und medizinischen Hilfsmitteln wie Bandagen und Kompressionsstrümpfen verwendet werden.

Erfindungsgemäß ist das Polymer ausgewählt aus der Gruppe bestehend aus Cellulose, modifizierter Cellulose, Modifikationen davon, Derivaten davon und Mi-schungen davon.

In einer offenbarten Ausführungsform ist das Polymer ausgewählt aus der Gruppe bestehend aus Cellulose, modifizierte Cellulose, Polyamid, Polyacryl, Polyester, Polyurethan, Polyethylen, Polypropylen, Modifikationen davon und Mischungen davon. In einer bevorzugten Ausführungsform ist das Polymer ausgewählt aus der Gruppe bestehend aus Cellulose, modifizierte Cellulose, Polyamid, Polyacryl, Polyester, Modifikationen davon und Mischungen davon.

In einer bevorzugten Ausführungsform ist das Polymer biologisch abbaubar. Das biologisch abbaubare Polymer ist bevorzugt ausgewählt aus der Gruppe bestehend aus Cellulose, modifizierter Cellulose, Latex, Eiweiß pflanzlicher sowie tierischer Herkunft sowie Gemischen davon. Auch können Polykondensations- und Polymerisations-Polymere, Polyurethane, Polyester, Polyacryle und Gemische von diesen Materialien verwendet werden.

In einer alternativen Ausführungsform kann jedoch auch vorgesehen sein, dass die erfindungsgemäße Polymerzusammensetzung auch nicht-biologisch abbaubare Polymere enthält. Beispiele solcher Polymere sind Polyamide, aromatische Polyamide, insbesondere Aramide, Polyacrylnitril, Polyester oder Polyvinylalkohole.

Erfindungsgemäß ist das Polymer Cellulose, modifizierte Cellulose, oder Modifikationen davon und Mischungen davon. In einer bevorzugten Ausführungsform ist das Polymer Cellulose oder modifizierte Cellulose.

Bei Cellulose handelt es sich um ein hydrophiles Netzwerk ausbildendes Polymer, dessen supramolekulare Strukturen sowohl in Lösung als auch im Festkörper über ebenfalls hydrophile Wasserstoff-Brückenbindungen stabilisiert sind. Dagegen sind die in der vorliegenden Erfindung verwendeten Wirkstoffe ausgewählt aus der Gruppe bestehend aus eine innere Okklusion erzeugende Wirkstoffe, eine äußere Okklusion erzeugende Wirkstoffe und Feuchtigkeitsspendende Wirkstoffe eher unpolare, lipophile organische Verbindungen. Es zeigte sich nun im Rahmen der vorliegenden Erfindung überraschenderweise, dass sich solche unpolaren, lipophilen Wirkstoffe in die cellulosischen Filamente und Fasern einbringen lassen. Ebenso überraschend ist es, dass die in dem hydrophilen Cellulosenetzwerk inkorporierten und polaren lipophilen organischen Wirkstoffe sich derart homogen und mikropartikulär verteilen lassen, dass eine feindosierbare und gleichmäßige Speicherung sowie kontrollierte Freisetzung dieser unpolaren lipophilen organischen Wirkstoffe aus den Fasern ermöglicht wird.

Bevorzugt wird die erfindungsgemäße Polymerzusammensetzung aus einer Spinnlösung hergestellt. Bevorzugt liegen die Wirkstoffe in der Polymerzusammensetzung als Mikroeinschlüsse vor.

Unter modifizierter Cellulose wird insbesondere Carboxyethyl-Cellulose, Methyl-Cellulose, Nitrat-Cellulose, Kupfer-Cellulose, Viskose, also Cellulose-Xantoghenat, Cellulosecarbamat und Celluloseacetat verstanden.

In einer alternativen Offenbarung ist das Polymer ein Polyamid, ein Polyacryl, ein Polyester oder Modifikationen davon und Mischungen davon. In einer Offenbarung ist das Polymer ein Polyamid, oder Modifikationen davon. In einer bevorzugten Ausführungsform ist das Polymer ein Polyacryl oder Modifikationen davon. In einer Offenbarung ist das Polymer ein Polyester oder Modifikationen davon.

Beispiele für Polykondensations- und Polymerisations-Polymere sind Polyamide, die zum Beispiel mit Methyl-, Hydroxi-, oder Benzylgruppen substituiert sind. Beispiele für Polyurethane sind solche, die auf der Basis von Polyether-Polyolen aufgebaut sind.

Erfindungsgemäß enthält die Polymerzusammensetzung mindestens einen Wirkstoff, wobei es sich bei dem Wirkstoff um einen eine innere Okklusion erzeugenden Wirkstoff oder einen eine äußere Okklusion erzeugenden Wirkstoff oder Mischungen hiervon handeln kann. Bevorzugt ist der mindestens eine Wirkstoff in die Polymerzusammensetzung inkorporiert und insbesondere nicht durch Tränkung oder Beschichtung auf die Trägermatrix aufgetragen.

Erfindungsgemäß enthält die Polymerzusammensetzung mindestens einen eine innere Okklusion erzeugenden Wirkstoff oder einen eine äußere Okklusion erzeugenden Wirkstoff.

Im Zusammenhang mit der vorliegenden Erfindung wird unter Okklusion die Verhinderung oder Reduzierung von Wasserverlust durch die Haut an die Umgebung, wie beispielsweise einer textilen Auflage, verstanden.

Neben weiteren wichtigen Funktionen schützt die menschliche Haut vor Angriffen aus der Umwelt. Zur Aufrechterhaltung ihrer Schutzfunktion ist es von wesentlicher Bedeutung, den Verlust von Wasser aus den tieferen Hautschichten zu vermeiden.

Der Aufbau der menschlichen Haut gliedert sich in drei Bestandteile, nämlich die Oberhaut (Epidermis), die Lederhaut (Dermis) und die Unterhaut (Subcutis). Eine besondere Rolle für den Wassergehalt der Haut spielen die äußeren Schichten des Stratum Corneum der Epidermis mit ihren Keratinfilamenten. So kann das Keratinfasergerüst beträchtliche Wassermengen halten. Ziel- und Angriffspunkt der äußeren okklusiven Wirkung eines entsprechenden Stoffes ist die Epidermis. Durch Aufbringen eines solchen Stoffes auf die Hautoberfläche entfaltet sich die Hauptwirkung, nämlich die Unterdrückung eines Entweichens von Wasser (trans-epidermaler Wasserverlust) aus dem Stratum Corneum. Durch diese Stoffe wird ein Film gebildet, der das Entweichen von Feuchtigkeit verhindert. Bei der äußeren Okklusion kann somit Wasser aus den tieferen Schichten nachrücken und das Keratinfasergerüst der äußeren Hautschicht mit Feuchtigkeit auffüllen. Dadurch schützt der Stratum Corneum vor Austrockung. Elastizität und Geschmeidigkeit der Haut werden erhalten und die Haut erhält ihr physiologisches Gleichgewicht zum Schutz beispielsweise vor Pilzbefall. Diese okklusive Wirkweise eines Wirkstoffes wird auch als äußere Okklusion bezeichnet

Die innere Okklusion bezeichnet eine Änderung der Hautlipidekonfiguration im Stratum Corneum. In der hexagonalen Konfiguration können die Kohlenwasserstoffketten der Lipide frei um ihre Achse rotieren. Diese Phase der "lockeren Packung" schafft eine durchlässigere, also permeable Struktur und erleichtert eine unerwünschte Wasserabgabe unter Stress im Stratum Corneum mit entsprechenden nachteiligen Folgen, wie beispielsweise Hauttrockenheit. Durch die Einwirkung bestimmter Wirkstoffe wird bei der inneren Okklusion eine dichtere ortho-rhombische Packung der Lipide erzeugt. Diese Anordnung führt zu einer festeren Struktur. Solch eine molekulare Aktion bewirkt durch die "ortho-rhombische Packung" der Lipide eine wesentlich geringere Durchlässigkeit der Hautbarriere und reduziert somit signifikant den Wasserverlust aus dem Stratum Corneum.

In einer Ausführungsform erzeugt der mindestens eine Wirkstoff eine innere Okklusion.

Bevorzugt ist der mindestens eine eine innere Okklusion erzeugende Wirkstoff ein Fettsäurederivat. Bevorzugt ist das Fettsäurederivat ausgewählt aus der Gruppe bestehend aus Isostearyl Isostearat, Isopropyl Palmitat und Mischungen davon. Bevorzugt ist das Fettsäurederivat Isostearyl Isostearat. Alternativ ist das Fettsäurederivat Isopropyl Palmitat.

In einer alternativen Ausführungsform erzeugt der mindestens eine Wirkstoff eine äußere Okklusion.

Bevorzugt ist der mindestens eine eine äußere Okklusion erzeugende Wirkstoff ausgewählt aus der Gruppe bestehend aus höhermolekulare Alkansäuren, höhermolekulare Alkensäuren, höhermolekulare Carbonate und Mischungen davon. Bevorzugt ist der mindestens eine eine äußere Okklusion erzeugende Wirkstoff ausgewählt aus der Gruppe bestehend aus Cetearyl Isononanoat, Dicaprylyl Carbonat und Mischungen davon. Bevorzugt ist der Wirkstoff Cetearyl Isononanoat. Bevorzugt ist der Wirkstoff Dicaprylyl Carbonat.

In einer alternativen Offenbarung ist der mindestens eine Wirkstoff ein feuchtigkeitsspendender Wirkstoff.

Bevorzugt ist der mindestens eine feuchtigkeitsspendende Wirkstoff ausgewählt aus der Gruppe bestehend aus Glycerin, Polyolen, insbesondere Panthenol, und anderen feuchtigkeitsspendenden Stoffen wie Harnstoff. Bevorzugt ist der mindestens eine feuchtigkeitsspendende Wirkstoff Glycerin (Propan-1,2,3-triol).

Bevorzugt ist der mindestens eine Schmerz oder Juckreiz reduzierenden Wirkstoff eine Fettsäureamidverbindung, eine Modifikationen davon oder ein Derivat davon oder ein Flavonoid, eine Modifikationen davon oder ein Derivat davon.

Bevorzugt ist der mindestens eine Schmerz oder Juckreiz reduzierenden Wirkstoff ausgewählt aus der Gruppe bestehend aus Palmitoylethanolamin (PEA), Stearoylethanolamid (SEA), Oxerutin, Troxerutin und Mischungen davon.

Bevorzugt ist der mindestens eine Schmerz oder Juckreiz reduzierenden Wirkstoff ein N-Acylethanolamin (NAE).

Bevorzugt ist der mindestens eine Schmerz oder Juckreiz reduzierenden Wirkstoff Palmitoylethanolamin (PEA) oder Stearoylethanolamid (SEA).

Die natürlich vorkommenden Fettamide PEA und seine Homologe wie das eng verwandte SEA weisen sehr gute anti-inflammatorische Eigenschaften auf. So werden sie als Lipid-Komponente in kosmetischen Formulierungen wie beispielsweise Haarwasch-Produkten gegen austrocknende Kopfhaut verwendet. Ein Einsatz dieser Stoffe erfolgt aber auch beispielsweise gegen Grippeviren, Erkältungskrankheiten, Erkrankungen der Atemwege und Rheumatisches Fieber. Auch werden die Stoffe als orale Gabe mit schmerzlindernder Eigenschaft verabreicht. Allgemein zeigen diese Stoffe eine Juckreiz mildernde, schmerzlindernde und entzündungshemmende Wirkung. Die menschliche Oberhaut kann sowohl durch schädliche Umwelteinflüsse als auch durch permanente Irritationen ausgelöst beispielsweise über textile Oberflächen angegriffen werden. Als Folge daraus entsteht die subjektive Empfindung von Juckreiz und Schmerz. Der physiologische Wirkmechanismus kann wie folgt erklärt werden: Schmerz- und Entzündungsreize (beispielsweise ausgelöst durch eine textile Oberfläche) treffen auf Rezeptoren der Schmerzweiterleitung und die Aktivierung der Entzündungs-Rezeptoren der Mastzellen führt zur Ausschüttung von stark entzündlich wirkenden Histaminen. Ergebnis ist zum einen die Auslösung der Schmerzempfindung durch Weiterleitung in den sensorischen Nervenfasern der Haut bis in die maßgeblichen Hirngebiete. Zum Zweiten führt die Ausschüttung der Entzündungsstoffe in der Epidermis zu einem Juckreiz, Rötungen und zu Trockenheit der Haut. Linderung auf molekularer Ebene schafft der Angriff von PEA und seinen Homologen aufgrund zweier Mechanismen: Zum einen handelt es sich bei PEA und seinen Homologen um einen Mastzell-Modulator. Die Histamin-Ausschüttung der Mastzellen wird durch erhöhte PEA-Konzentrationen am Ort der Entzündung gehemmt. Zum anderen haben PEA und seine Homologe eine hohe Affinität zu den für Schmerz und Entzündung verantwortlichen Rezeptoren. Dies führt zu einer Unterbindung der entsprechenden Reizleitung. So zeigen PEA und seine Homologe bei Einwirkung auf betroffene Hautgebiete eine entzündungshemmende, beruhigende Wirkung für trockene juckende und gereizte Haut.

Bevorzugt ist der mindestens eine Schmerz oder Juckreiz reduzierenden Wirkstoff ein Flavonoid, eine Modifikationen davon oder ein Derivat davon. Bevorzugt ist der mindestens eine Schmerz oder Juckreiz reduzierenden Wirkstoff ein Rutin, eine Modifikationen davon oder ein Derivat davon.

Bevorzugt ist der mindestens eine Schmerz oder Juckreiz reduzierenden Wirkstoff Rutin, Oxerutin oder Troxerutin, eine Modifikationen davon oder ein Derivat davon. Bevorzugt ist der mindestens eine Schmerz oder Juckreiz reduzierenden Wirkstoff Troxerutin, eine Modifikationen davon oder ein Derivat davon. Bevorzugt ist der mindestens eine Schmerz oder Juckreiz reduzierenden Wirkstoff Troxerutin.

Troxerutin ist ein pflanzlicher Wirkstoff aus der Gruppe der Flavonoide und wird bei Krankheiten eingesetzt, die mit einer erhöhten Durchlässigkeit der Blutgefäße einhergehen. Zu nennen sind hier Venenerkrankungen, die mit Wassereinlagerungen im Gewebe, also Ödemen einhergehen. Dazu gehören beispielsweise oberflächliche Venenentzündungen oder Krampfadern sowie die Venenschwächen während der Schwangerschaft. Auch bei offenen Beinen, Chronischvenöser Insuffizienz und bei schweren, müden Beinen oder Beinkrämpfen, die auf eine Verstopfung der Beinvenen zurückzuführen sind, werden Rutine wie beispielsweise Troxerutin angewandt. Bei Stauungen in den Venen entstehen neben einer Schädigung der Gefäßinnenwände Blutklümpchen, Verstopfungen und Entzündungen. Gefäßinnenwandsschädigungen werden durch Stoffe verursacht, die die weißen Blutkörperchen im Rahmen der Entzündung freisetzen. Troxerutin verhindert die Abgabe dieser Stoffe und stabilisiert so die durchlässigen Gefäßwände der Blutkapillaren. Diese werden derart abgedichtet, dass keine Flüssigkeit mehr austreten kann. Außerdem verbessert Troxerutin die Fließeigenschaften des Blutes, indem es die roten Blutkörperchen verformbarer macht und eine leicht gerinnungshemmende Wirkung hat. Die Summe dieser Effekte trägt dazu bei, dass bei Blutstauungen in den Venen weniger Wasser in das umliegende Gewebe austritt und Ödeme bildet. Symptome der Venenerkrankungen wie Schmerzen und Schweregefühl in den Beinen werden auf diese Art durch die Anwendung von Troxerutin gelindert.

In einer bevorzugten Ausführungsform enthält die Polymerzusammensetzung mindestens einen eine innere Okklusion erzeugenden Wirkstoff oder einen eine äußere Okklusion erzeugenden Wirkstoff und mindestens einen Schmerz oder Juckreiz reduzierenden Wirkstoff.

In einer Offenbarung enthält die Polymerzusammensetzung mindestens einen feuchtigkeitsspenden Wirkstoff und mindestens einen Schmerz oder Juckreiz reduzierenden Wirkstoff.

In einer bevorzugten Ausführungsform enthält die Polymerzusammensetzung mindestens einen eine innere Okklusion erzeugenden Wirkstoff oder einen eine äußere Okklusion erzeugenden Wirkstoff und mindestens einen feuchtigkeitsspendenden Wirkstoff.

In einer bevorzugten Ausführungsform enthält die Polymerzusammensetzung mindestens einen eine innere Okklusion erzeugenden Wirkstoff oder einen eine äußere Okklusion erzeugenden Wirkstoff, mindestens einen Schmerz oder Juckreiz reduzierenden Wirkstoff und mindestens einen feuchtigkeitsspendenden Wirkstoff.

Die vorliegende Offenbarung betrifft auch einen Formkörper oder eine Vielzahl von Formkörpern umfassend eine erfindungsgemäße Polymerzusammensetzung.

Ein Vorteil der offenbarten Formkörper, in denen die erfindungsgemäßen Additive enthalten sind, ist der gleichmäßige Einbau der Wirkstoffe in dem Formkörper, insbesondere in die Fasermatrix, bei unterschiedlich herstellbaren Faserquerschnitten. Dabei ist auch die Verarbeitung als Monofilament- oder Endlosfilamentgarn möglich, wodurch sich eine Einsatzmöglichkeit bei technischen Artikeln ergibt.

Die offenbarten Formkörper können für verschiedenste Zwecke verwandt werden.

Bevorzugt ist der Formkörper eine Faser. Bevorzugt ist die Faser eine Stapelfaser, ein Monofilament, ein multifiles Filament oder ein multifiles Endlosfilament.

Insbesondere wenn die Faser eine cellulosische Faser ist, kann sie beispielsweise wie in den Figuren 1 und 2 der DE 10 2007 054 702 A1 gezeigt aufgebaut sein. Der mindestens eine Wirkstoff kann in Form von dispergierten Einschlüssen in einer Matrix, insbesondere Cellulosematrix vorliegen.

Bevorzugt ist der mindestens eine Wirkstoff mit zumindest einem hydrophoben Verdickungsmittel stabilisiert.

Insbesondere, wenn aus den erfindungsgemäßen Fasern eine textile Fläche hergestellt wird, kann dieses nicht nur aus der erfindungsgemäßen Faser bestehen, sondern auch zusätzliche Komponenten enthalten, beispielsweise Baumwolle, Lyocell, Rayon, Carbacell, Polyester, Polyamid, Celluloseacetat, Acrylat, Polypropylen oder Gemische davon.

Die vorliegende Erfindung betrifft auch die Verwendung eines erfindungsgemäßen Formkörpers zur Herstellung von Garnen.

Die vorliegende Erfindung betrifft ein Garn, enthaltend einen erfindungsgemäßen Formkörper. Die vorliegende Erfindung betrifft auch ein Garn, enthaltend die erfindungsgemäßen Formkörper als eine Grundkomponente. Die vorliegende Erfindung betrifft auch ein Garn, bestehend aus erfindungsgemäßen Formkörpern.

Die vorliegende Erfindung betrifft auch einen Vliesstoff, enthaltend einen erfindungsgemäßen Formkörper. Die vorliegende Erfindung betrifft auch einen Vliesstoff, enthaltend ein erfindungsgemäßes Garn.

Die vorliegende Erfindung betrifft auch ein Kleidungstück oder ein medizinisches Hilfsmittel, enthaltend einen erfindungsgemäßen Formkörper oder ein erfindungsgemäßes Garn. Bevorzugt handelt es sich um Kleidungsstücke oder medizinische Hilfsmittel, die beim Tragen zumindest teilweise direkt an der Haut anliegen.

Die vorliegende Erfindung betrifft auch ein Kleidungstück, enthaltend einen erfindungsgemäßen Formkörper oder ein erfindungsgemäßes Garn. Bevorzugt ist das Kleidungsstück Unterwäsche, insbesondere eine Unterhose, ein Unterhemd oder ein Strumpf. Alternativ kann es sich bei dem Kleidungsstück auch um einen Handschuh handeln.

Die vorliegende Erfindung betrifft auch ein medizinisches Hilfsmittel, enthaltend einen erfindungsgemäßen Formkörper oder ein erfindungsgemäßes Garn. Bevorzugt handelt es sich bei dem medizinischen Hilfsmittel um einen Kompressionsstrumpf oder um eine Bandage.

Die vorliegende Erfindung betrifft auch eine Sportbandage und andere Sporthilfsmittel, enthaltend einen erfindungsgemäßen Formkörper oder ein erfindungsgemäßes Garn.

Die vorliegende Offenbarung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Pflege der Haut.

Die vorliegende Offenbarung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Verhinderung des Austrocknens der Haut.

Die vorliegende Offenbarung betrifft auch die Verwendung einer erfindungsgemäßen Polymerzusammensetzung oder eines erfindungsgemäßen Formkörpers in einem Kleidungstück, einem Sporthilfsmittel oder in einem medizinischen Hilfsmittel zur Reduzierung von Schmerz und/oder Juckreiz, insbesondere bei geschädigter Haut und bei Hautkrankheiten, wie beispielsweise bei Neurodermitis. Bevorzugt wird dabei gleichzeitig ein Austrocknen der Haut reduziert oder verhindert.

## Patentansprüche

1. Garn, enthaltend einen Formkörper umfassend eine Polymerzusammensetzung, enthaltend ein Polymer und mindestens einen Wirkstoff ausgewählt aus der Gruppe bestehend aus einem eine innere Okklusion erzeugenden Wirkstoff oder einem eine äußere Okklusion erzeugenden Wirkstoff und Mischungen davon, wobei das Polymer ausgewählt ist aus der Gruppe bestehend aus Cellulose, modifizierter Cellulose, Modifikationen davon, Derivaten davon und Mischungen davon.

2. Garn nach Anspruch 1, wobei der mindestens eine eine innere Okklusion erzeugende Wirkstoff ausgewählt ist aus der Gruppe bestehend aus Isostearyl Isostearat, Isopropyl Palmitat und Mischungen davon

3. Garn nach einem der vorhergehenden Ansprüche, wobei der mindestens eine eine äußere Okklusion erzeugende Wirkstoff ausgewählt ist aus der Gruppe bestehend aus Cetearyl Isononanoat, Dicaprylyl Carbonat und Mischungen davon.

4. Garn nach einem der vorhergehenden Ansprüche, wobei das Polymer Cellulose ist.

5. Garn nach einem der vorhergehenden Ansprüche, wobei der Formkörper eine Faser ist.

6. Garn nach Anspruch 5, wobei die Faser eine Stapelfaser, ein Monofilament, ein multifiles Filament oder ein multifiles Endlosfilament ist.

7. Verwendung eines Formkörpers zur Herstellung von Garnen, wobei der Formkörper eine Polymerzusammensetzung umfasst, enthaltend ein Polymer und mindestens einen Wirkstoff ausgewählt aus der Gruppe bestehend aus einem eine innere Okklusion erzeugenden Wirkstoff oder einem eine äußere Okklusion erzeugenden Wirkstoff und Mischungen davon, wobei das Polymer ausgewählt ist aus der Gruppe bestehend aus Cellulose, modifizierter Cellulose, Modifikationen davon, Derivaten davon und Mischungen davon.

8. Verwendung nach Anspruch 7, wobei der mindestens eine eine innere Okklusion erzeugende Wirkstoff ausgewählt ist aus der Gruppe bestehend aus Isostearyl Isostearat, Isopropyl Palmitat und Mischungen davon und wobei der mindestens eine eine äußere Okklusion erzeugende Wirkstoff ausgewählt ist aus der Gruppe bestehend aus Cetearyl Isononanoat, Dicaprylyl Carbonat und Mischungen davon.

9. Kleidungstück oder medizinisches Hilfsmittel, enthaltend einen Garn nach einem der Ansprüche 1 bis 6.

10. Kleidungstück nach Anspruch 9, wobei das Kleidungstück eine Unterhose, ein Unterhemd oder ein Strumpf ist.

11. Medizinisches Hilfsmittel nach Anspruch 9, wobei das medizinische Hilfsmittel ein Kompressionsstrumpf oder eine Bandage ist.

12. Sportbandage, enthaltend einen Garn nach einem der Ansprüche 1 bis 6.

13. Verwendung eines Garns nach einem der Ansprüche 1 bis 6 in einem Kleidungstück, in einem Sporthilfsmittel oder in einem medizinischen Hilfsmittel zur Pflege der Haut.

## Claims

1. A yarn containing a formed body comprising a polymer composition, containing a polymer and at least one agent selected from the group consisting of an agent generating an internal occlusion or an agent generating an external occlusion and mixtures thereof, wherein the polymer is selected from the group consisting of cellulose, modified cellulose, modifications thereof, derivates thereof and mixtures thereof.

2. The yarn according to claim 1, wherein the at least one agent generating an internal occlusion is selected from the group consisting of isostearyl isostearate, isopropyl palmitate and mixtures thereof.

3. The yarn according to any one of the preceding claims, wherein the at least one agent generating an external occlusion is selected from the group consisting of cetearyl isononanoate, dicaprylyl carbonate and mixtures thereof.

4. The yarn according to any one of the preceding claims, wherein the polymer is cellulose.

5. The yarn according to any one of the preceding claims, wherein the formed body is a fibre.

6. The yarn according to claim 5, wherein the fibre is a staple fibre, a monofilament, a multifilament or a continuous multifilament.

7. A use of a formed body for the fabrication of yarns, wherein the formed body comprises a polymer composition containing a polymer and at least one agent selected from the group consisting of an agent generating an internal occlusion or an agent generating an external occlusion and mixtures thereof, wherein the polymer is selected from the group consisting of cellulose, modified cellulose, modifications thereof, derivates thereof and mixtures thereof.

8. The use according to claim 7, wherein the at least one agent generating an internal occlusion is selected from the group consisting of isostearyl isostearate, isopropyl palmitate and mixtures thereof, and wherein the at least one agent generating an external occlusion is selected from the group consisting of cetearyl isononanoate, dicaprylyl carbonate and mixtures thereof.

9. A clothing article or medical aid containing a yarn according to any one of claims 1 to 6.

10. The clothing article according to claim 9, wherein the clothing article is a pair of underpants, an undershirt or a stocking.

11. A medical aid according to claim 9, wherein the medical aid is a compression stocking or a bandage.

12. A sport bandage containing a yarn according to any one of claims 1 to 6.

13. A use of a yarn according to any one of claims 1 to 6 in an article of clothing, in a sporting aid or in a medical aid for skin care.

## Revendications

1. Fil contenant un corps façonné comprenant une composition de polymère, contenant un polymère et au moins un agent actif choisi dans le groupe consistant en un agent actif effectuant une occlusion interne ou un agent actif effectuant une occlusion externe, et leurs mélanges, dans lequel le polymère est choisi dans le groupe consistant en la cellulose, la cellulose modifiée, leurs modifications, leurs dérivés et leurs mélanges.

2. Fil selon la revendication 1, dans lequel le au moins un agent actif effectuant une occlusion interne est choisi dans le groupe consistant en l'isostéarate d'isostéaryl, le palmitate d'isopropyl et leurs mélanges.

3. Fil selon l'une quelconque des revendications précédentes, dans lequel le au moins un agent actif effectuant une occlusion externe est choisi dans le groupe consistant en l'isononanoate de cétéaryle, le carbonat de dicaprylyle et leurs mélanges.

4. Fil selon l'une quelconque des revendications précédentes, dans lequel le polymère est la cellulose.

5. Fil selon l'une quelconque des revendications précédentes, dans lequel le corps façonné est une fibre.

6. Fil selon la revendication 5, dans lequel la fibre est une fibre coupée, un monofilament, un multifilament ou un multifilament sans fin.

7. Utilisation d'un corps façonné pour la fabrication des fils, dans laquelle le corps façonné comprend une composition de polymère contenant un polymère et au moins un agent actif choisi dans le groupe consistant en un agent actif effectuant une occlusion interne ou un agent actif effectuant une occlusion externe et leurs mélanges, dans lequel le polymère est choisi dans le groupe consistant en la cellulose, la cellulose modifiée, leurs modifications, leurs dérivés et leurs mélanges.

8. Utilisation selon la revendication 7, dans lequel le au moins un agent actif effectuant une occlusion interne est choisi dans le groupe consistant en l'isostéarate d'isostéaryl, le palmitate d'isopropyl et leurs mélanges, et dans lequel le au moins un agent actif effectuant une occlusion externe est choisi dans le groupe consistant en l'isononanoate de cétéaryle, le carbonat de dicaprylyle et leurs mélanges.

9. Article d'habillement ou d'aide médicale comprenant un fil selon l'une quelconque des revendications 1 à 6.

10. Article d'habillement selon la revendication 9, dans lequel l'article d'habillement est une culotte, un maillot de corps ou un bas.

11. Aide médicale selon la revendication 9, dans lequel l'aide médicale est un bas de compression ou un bandage.

12. Bandage de sport, contenant un fil selon l'une quelconque des revendications 1 à 6.

13. Utilisation d'un fil selon l'une quelconque des revendications 1 à 6 dans un article d'habillement, dans une aide de sport ou dans une aide médicale pour les soins de la peau.
